# EUROPEAN PATENT APPLICATION

(11) **EP 4 729 629 A1**
(43) Date of publication of application: **22.04.2026**
(21) Application number: 24206662.9
(22) Date of filing: 15.10.2024
(51) Int. Cl.: C12Q 1/6839

(54) **A LABEL, MARKER AND A METHOD FOR ANALYSING A BIOLOGICAL SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

In a first aspect, a label (102, 402, 600) for analysing a biological sample is provided. The label (102, 402, 600) comprises a first label part (300) comprising at least one first nucleic acid strand (104, 302), a second label part comprising a second nucleic acid strand (108), and a third label part comprising a third nucleic acid strand (110). The first nucleic acid strand (104, 302), the second nucleic acid strand (108), and the third nucleic acid strand (110) are configured to form a triplex with each other. The first label part (300) further comprises at least one labelling moiety (106, 304, 306). In further aspects, a respective marker (100, 400, 500) and method for analysing a biological sample are provided.

## Description

### Technical field

The invention relates to a label and marker for analysing a biological sample. The label and marker comprise nucleic acid strands configured to form a triplex. In a further aspect, a respective method for analysing a biological sample is provided.

### Background

Labels and markers are frequently used when analysing biological samples, for example in fluorescent microscopy. Markers for fluorescent microscopy generally comprise affinity reagents, such as antibodies, and labels attached to the affinity reagents, in order to enable specifically attaching the labels- in particular via the affinity reagents - to a target analyte in a biological sample. This allows the identification and/or localisation of the target analyte in the biological sample.

The various applications of markers include multiplexing approaches, which require large sets of distinguishable markers, whilst detecting analytes that only occur in small quantities requires markers with bright labels. It is of constant interest to provide markers and labels for microscopy applications that allow flexible use and high reliability when detecting and identifying target analytes.

### Summary

It is an object to provide labels and markers for analysing biological samples that are flexible to use and easy to assemble.

The aforementioned object is achieved by the subject-matter of the independent claims. Advantageous embodiments are defined in the dependent claims and the following description.

In a first aspect, a label for analysing a biological sample is provided. The label comprises a first label part comprising at least one first nucleic acid strand, a second label part comprising a second nucleic acid strand, and a third label part comprising a third nucleic acid strand. The first nucleic acid strand, the second nucleic acid strand, and the third nucleic acid strand are preferably single stranded nucleic acids. The first nucleic acid strand, the second nucleic acid strand, and the third nucleic acid strand are configured to form a triplex with each other. The first label part further comprises at least one labelling moiety.

In particular, at least one of the first nucleic acid strand, the second nucleic acid strand, and the third nucleic acid strand, preferably the second nucleic acid strand and/or the third nucleic acid strand, may be an endogenous nucleic acid of the biological sample, such as an RNA or a DNA of the biological sample. In this case, the remaining nucleic acid strands (preferably the first) are exogenous.

Preferably, all of the first, second, and third nucleic acids are exogenous nucleic acids. Thus, they are not nucleic acids of the biological sample, but they are introduced into the biological sample when analysing the sample. This is further explained below in connection with the method.

Generally, exogenous nucleic acids are specifically (chemically) synthesised for the label or to generate the label, preferably not synthesised in or by the biological sample. In contrast, endogenous nucleic acids may be characterised by having been synthesised by or in the biological sample.

The nucleic acid triplex is formed of the three oligonucleotides, in particular of the first nucleic acid strand, the second nucleic acid strand, and the third nucleic acid strand winding around each other. Thus, the triplex is a triple helix, in particular. In the triplex, the second nucleic acid strand and the third nucleic acid strand are preferably configured to form a duplex, in particular a double helix structure, such as B-form DNA, via Watson and Crick base-pairing. The first nucleic acid strand is configured to bind to the duplex to form the triplex, in particular via Hoogsteen base-pairing. Thus, in particular, the label comprises a triplex structure of the first nucleic acid strand, the second nucleic acid strand, and the third nucleic acid strand.

The first nucleic acid strand preferably does not hybridise to the second nucleic acid strand and/or the third nucleic acid strand based on Watson and Crick base-pairing.

The at least one labelling moiety may be covalently attached to the first strand. In particular, the labelling moiety may be configured to be optically detectable. For example, the labelling moiety may be a fluorophore, such as a fluorescent protein, an organic or inorganic fluorescent molecule, or a fluorescent nanoparticle. This renders the label detectable by an optical microscope, such as a fluorescence microscope, in particular a confocal scanning microscope, for example. The labelling moiety may be a fluorophore or fluorescent dye. For example, the labelling moiety may be ATTO 390, ATTO 425, ATTO 488, ATTO 495, ATTO490LS, ATTO 550, ATTO 565, ATTO 594, ATTO 647N, or ATTO700. Other example fluorescent dyes include Alexa Fluor dyes, CF dyes, BODIPY dyes, or Cy dyes, which may be equally suitable.

The label may be used for determining the proximity of the second label part and the third label part or the simultaneous presence of both the second label part and the third label part in the biological sample. In particular, the first label part only binds to the duplex of the second label part and the third label part. Thus, since hybridising requires proximity or conversely hybridisation results in proximity of the second label part and the third label part, the first label part can be used to determine the proximity between the second and third label part or their presence in the biological sample.

Preferably, the second nucleic acid strand and the third nucleic acid strand are at least partially complementary to each other, in particular based on Watson-Crick base pairing. Thus, the second and the third nucleic acid strands form the duplex with each other. This enables stable hybridisation of the second nucleic acid strand and the third nucleic acid strand to each other. In particular, the second nucleic acid strand and the third nucleic acid strand have respective sequences that are at least partially complementary to each other.

Preferably, the at least one first nucleic acid strand is at least partially complementary to the hybridised second nucleic acid strand and third nucleic acid strand, in particular based on Hoogsteen base-pairing. Thus, the first nucleic acid strand can hybridise to the duplex of the second nucleic acid strand and the third nucleic acid strand, in particular to a major groove of the duplex. This enables a stable triplex. In particular, the first nucleic acid strand has a respective sequence that is at least partially complementary to the duplex of the second nucleic acid strand and the third nucleic acid strand.

Preferably, the at least one first nucleic acid strand is a nucleic acid analogue, such as a peptide nucleic acid (PNA). In this case, the second and third nucleic acid strands are preferably natural nucleic acids. This enables a robust first label part and a stable triplex. The peptide nucleic acid is particularly preferred since it does not have a negatively charged phosphate backbone and therefore has a particular high affinity to the duplex.

Generally, nucleic acid analogues are compounds which are structurally similar to naturally occurring RNA and (D-)DNA. Nucleic acids are chains of nucleotides, which are composed of three parts: a phosphate backbone, a pentose sugar, either ribose or deoxyribose, and one of four nucleobases. An analogue may have any of these altered. The nucleic acid analogue may be an artificial nucleic acid or a xeno nucleic acid, such as PNA, or L-DNA.

Preferably, at least one of the first nucleic acid strand, the second nucleic acid strand, and the third nucleic acid strand is a nuclease-resistant nucleic acid and/or wherein at least one of the other of the first nucleic acid strand, the second nucleic acid strand, and the third nucleic acid strand is a nuclease-sensitive nucleic acid. Whereas natural nucleic acids or naturally occurring nucleic acids (DNA and RNA) are generally sensitive to degradation agents such as nucleases, nucleic acid analogues are generally resistant to degradation agents such as nucleases. Thus, the nuclease-sensitive nucleic acid may be a naturally occurring nucleic acid and the nuclease-resistant nucleic acid may be a nucleic acid analogue. In a particular embodiment, the first nucleic acid strand is a nuclease-sensitive nucleic acid and the second nucleic acid strand and the third nucleic acid strand are nuclease-resistant nucleic acids. This enables selectively removing the first nucleic acid strand with the labelling moiety, for example, when performing iterative or cyclical staining methods.

Preferably, the label comprises a plurality of first nucleic acid strands that each bind to a different part of the hybridised second nucleic acid strand and third nucleic acid strand. In particular, each first nucleic acid strand may comprise between 5 and 25 nucleotides. Thus, each first nucleic acid strand may bind to a different section of the duplex formed between the second and third nucleic acid strand. Since the degree or length of duplex formation between the second and third nucleic acid strand may depend on the distance between the second and third nucleic acid strands (for example, if the label is used as a proximity assay, in this case the second nucleic acid strand and the third nucleic acid strand may only partially hybridise to each other), a varying number of first nucleic acid strands may bind to the duplex. This enables correlating a detection signal of the number of labelling moieties associated with the duplex via the first nucleic acid strands to the distance between the second and third nucleic acid strands.

Preferably, the at least one first nucleic acid strand comprises an affinity moiety. For example, the affinity moiety may be biotin, streptavidin, adamantane, or ferrocene. This enables efficiently binding the first label part to a surface of a glass slide, a surface of a lateral flow device, a surface of a semiconductor electrode, or to a stationary phase of a chromatography column. To that end, the surface or the stationary phase may comprise a respective second affinity moiety, such as streptavidin or biotin, or a cucurbituril molecule, in order to attach the first label part.

In a particular embodiment, at least two of the first label part, the second label part and the third label part of the label may each comprise an affinity moiety. In particular, the affinity moieties may be covalently attached to the respective nucleic acid strand of the label parts. This enables immobilising the respective label part.

Preferably, the at least one first nucleic acid strand is immobilised on, in particular attached to, a solid support. For example, the first nucleic acid may be immobilised by means of the affinity moiety. The solid support may be a surface of a glass slide, a lateral flow device, a semiconductor electrode, or a stationary phase of a chromatography column. Thus, this enables separating the first label part, in particular, the label with all label parts, from any other components of the biological sample. The third label part may alternatively be removed from the triplex and detected separately, by means of the affinity moiety.

Alternatively, the affinity moiety may be an antibody, a nanobody, a fragment of an antibody (e.g. Fab fragment), an aptamer, a darpin, or an artificial polymeric binder, each of the aforementioned being configured to specifically bind to a target analyte in the sample, an oligonucleotide probe, a drug or drug-like molecule, or a toxin. An antibody may be easily immobilized by using Protein A, G, or A/G.

Preferably, the at least one first nucleic acid strand is configured to form a secondary structure, in particular, wherein the secondary structure is configured to unfold when the triplex is formed. For example, the secondary structure may be a stem-loop or a hairpin loop of the first nucleic acid strand. To that end, the first nucleic acid strand may comprise a first nucleotide sequence and a second nucleotide sequence, wherein the second nucleotide sequence is a reverse complement of the first nucleotide sequence. In particular in this case, the first nucleic acid strand may further comprise at least two labelling moieties, which are (covalently) attached to opposing ends of the first nucleic acid strand. The at least two labelling moieties may be configured for non-radiative energy transfer between them, for example, one of the at least two labelling moieties may quench the fluorescent emission of the other of the at least two labelling moieties. In this case, the first label part may also be called a molecular beacon.

Preferably, the label further comprises at least one guest molecule configured to form a complex with a host molecule. This enables controlling the formation of the duplex and/or the triplex. In particular, the at least one guest molecule is (covalently) attached to one of the first nucleic acid strand, the second nucleic acid strand, or the third nucleic acid strand, for example, their respective phosphate backbone or nucleobase. Preferably, the guest molecule is (covalently) attached to the second nucleic acid strand and/or the third strand. The guest molecule enables disrupting or hindering duplex and/or triplex formation, in particular when a guest-host complex is formed.

The guest molecule is preferably covalently attached to one of the nucleic acid strands. In particular, the label may comprise a plurality of guest molecules that are attached to at least one of the nucleic acid strands. In this case, one guest molecule may bind to one host molecule. In particular, the at least one guest molecule is not a nucleotide and/or not a labelling moiety. In a particular embodiment, there may be one guest molecule for every ten nucleotides of one of the nucleic acid strand.

The guest molecule is preferably configured to selectively form a complex with the host molecule. Thus, the guest molecule may be configured to form the complex with the host molecule only under a particular complexing condition. Similarly, the guest molecule may be configured to decomplex from the host molecule only under a decomplexing condition, which is different to the complexing condition. In the simplest case the complexing condition is characterised by presence or a high concentration (e.g. µM-mM range) of host molecule and the decomplexing condition is characterized by a low concentration or absence of the host molecule. The complex formation may include binding of the guest molecule to the host molecule. For example, the complex formation may be based on intermolecular forces such as hydrogen bonding, Van der Waals forces, or dipole interactions. Preferably, the complex formed between the host molecule and the guest molecule is configured to disrupt or hinder hybridisation of the respective nucleic acid strands to each other. In particular, the complex may reduce the melting temperature of the duplex of the hybridised second and third nucleic acid strand.

Preferably, the at least one guest molecule is configured to form the complex with the host molecule under a complexing condition. Similarly, the guest molecule and/or the host molecule are configured to decomplex under a decomplexing condition. Such a decomplexing condition and/or complexing condition may include varying a concentration of the host molecule or addition of a competitor guest molecule, varying pH, salt, and/or temperature. This enables selectively forming the complex between the host molecule and the guest molecule. In particular, the complexation of the at least one guest molecule with the host molecule may lead to a strand invasion and a dissociation of the duplex between the second and third nucleic acid strand.

Preferably, the at least one guest molecule is one of 1-adamantanemethylamine, ferrocenyl methylamine, 1,4-benzenedimethanamine, and 4-tertbutylbenzylamine. Xiao et al, 2022 (Controllable DNA hybridization by host-guest complexation-mediated ligand invasion. Nature Communications 13: 5936) provides further details of suitable host- and guest-molecules.

Preferably, the label comprises at least one host molecule. Providing the label with the at least one guest molecule and host molecule enables avoiding random or spontaneous hybridisation of the nucleic acid strands. In particular, this enables controlling the hybridisation between the second and third nucleic acid strands. This makes the introduction of the label parts into a biological sample easier and more efficient. In particular, the label may comprise a plurality of host molecules to at least form a complex with each guest molecule. The host molecule may be configured to form a complex with the guest molecule.

Preferably, the host molecule is a cucurbit[n]uril, in particular cucurbit[7]uril.

Preferably, the label further comprises at least one blocking nucleic acid strand at least partially complementary to one of the second nucleic acid strand and the third nucleic acid strand. Thus, the blocking nucleic acid strand may form a duplex with the respective second or third nucleic strand. The blocking strand enables control over the hybridisation between the second and third nucleic acid strands. This makes the introduction of the label parts into a biological sample easier and more efficient. The blocking strand may be removed from the respective second or third nucleic acid strand once the label parts have been introduced into the biological sample. For example, the hybridised complex of the blocking strand and the respective second or third nucleic acid strand may have a lower melting temperature than the second and third nucleic acid strand. Alternatively, the first, second and third nucleic acid strands may be a nuclease-resistant nucleic acid and the blocking strand may be nuclease-sensitive, enabling the blocking strand removal by addition of a suitable nuclease.

The blocking strand may also have an attached guest molecule, for example. This similarly enable control over its hybridisation depending on the presence of the respective host molecule.

In a further aspect, a marker for analysing a biological sample with a plurality of target analytes is provided. The marker comprises a label, in particular as described above, comprising a first label part, a second label part and a third label part. The marker comprises a first marker part comprising a first affinity reagent and the second label part, in particular, the second nucleic acid strand is (covalently) attached to the first affinity reagent. The marker further comprises a second marker part comprising a second affinity reagent and the third label part, in particular, the third nucleic acid strand is (covalently) attached to the second affinity reagent. The first affinity reagent and the second affinity reagent are each configured to bind specifically to one of the target analytes of the biological sample.

In particular, the first affinity reagent and the second affinity reagent may be configured to bind specifically to different target analytes of the biological sample. Alternatively, the first affinity reagent and the second affinity reagent may be configured to bind specifically to the same target analyte, however, to different regions or epitopes of the same target analyte.

Each of the first affinity reagent and the second affinity reagent may be one of an antibody, an antibody fragment, an aptamer, and a linear or primary nucleic acid.

The marker enables specifically attaching label parts to target analytes of the biological sample. By means of the first label part, the first marker part and the second marker part may be determined to be in close proximity. In particular, the respective target analytes of the first and second marker parts may be determined to be in close proximity.

In another aspect, a method for analysing a biological sample is provided. The method comprises the step of introducing at least a first label part, in particular as described above for the label, into the biological sample. The first label part comprises at least one first nucleic acid strand and at least one labelling moiety. The first nucleic acid strand is configured to form a triplex with a second nucleic acid strand and a third nucleic acid strand. The method further comprises the step of generating an optical readout of the biological sample with at least the first label part.

In particular, the method enables efficiently determining whether the second nucleic acid strand and the third nucleic acid strand are present in the biological sample. By using the first nucleic acid strand having a sequence that forms a triplex with the second nucleic acid strand and the third nucleic acid strand, the first label part hybridises specifically to the second nucleic acid strand and the third nucleic acid strand and their presence may be determined in the biological sample, in particular in an optical readout, for example generated by means a microscope. In this case, at least one of the second nucleic acid strand and the third nucleic acid strand may be an endogenous nucleic acid of the biological sample and the target analyte. In any case, the first nucleic acid strand may be an exogenous nucleic acid, that is not of the biological sample.

The second nucleic acid strand and the third nucleic acid strand may be configured to hybridise to each other and to form a duplex. The first nucleic acid strand may hybridise to the duplex, forming the triplex. Thus, the first, second, and third nucleic acids may be configured to form the triplex.

The optical readout enables determining presence of a target analyte. The optical readout may be generated by means of a microscope, for example a fluorescence microscope. Alternatively or in addition, the first nucleic acid strand may be connected to an electrode in order to detect the presence of the marker, for example, by means of the affinity moiety.

In particular, the first label part may be supplemented with the features described for the label above.

Preferably, at least one of the second nucleic acid strand and the third nucleic acid strand are an endogenous nucleic acid of the biological sample. An endogenous nucleic acid is part of the biological sample, in particular prior to the start of the method. An example of an endogenous nucleic acid is an mRNA generated in a cellular biological sample. The method therefore enables detecting the endogenous nucleic acid with high specificity since several specific affinity interactions are required to form the triplex and generate a detectable signal. In case only one of the second and third nucleic acid strands is endogenous, the other nucleic acid strand may be introduced for example with the first label part and therefore be an exogenous nucleic acid.

Preferably, the method comprises a step of introducing one of the second nucleic acid strand and the third nucleic acid strand into the biological sample. In this case, the nucleic acid introduced into the biological sample is an exogenous nucleic acid and the other one is an endogenous nucleic acid. In particular, the one of the second nucleic acid strand and the third nucleic acid strand is introduced together with the first label part.

In a particular preferred embodiment, a second label part comprises the second nucleic acid strand and a third label part comprises the third nucleic acid strand, and the first label part, the second label part and the third label part are of a marker, in particular as described above, and the marker is introduced into the biological sample. In particular, this is an alternative embodiment to the embodiments described above that use endogenous nucleic acids. For the present embodiment, all of the first nucleic acid strand, the second nucleic acid strand and the third nucleic acid strand are exogenous nucleic acids. The first label part, the second label part and the third label part are preferably of a label according to one of the embodiments described above.

The label parts may be introduced separately or sequentially to the biological sample, in particular, the third label part is introduced last. Thus, the method may have the steps of introducing a marker into the biological sample and generating an optical readout of the biological sample with the marker.

Preferably, the method comprises the steps of removing the first label part from the second nucleic acid strand and third nucleic acid strand, and of introducing a further first label part with a further first nucleic acid strand configured to form a triplex with the second nucleic acid strand and the third nucleic acid strand. This enables a cyclical staining, for example for high-plex applications, where a large number of target analytes is sequentially marked. In particular, the first label part comprises at least one further labelling moiety, in particular with optical properties that differ from the optical properties of the labelling moiety of the first label part. The method may further comprise a further optical readout of the biological sample with the further first label part.

In case any of the first, second and third nucleic acid strands used in the method comprises a guest molecule, the respective nucleic acid strand may preferably be introduced into the biological sample in the presence of the host molecule to form the guest-host complex. The method may preferably further comprise a step of applying a decomplex condition in order to remove the host molecule from the guest molecule and to enable hybridisation to form the duplex and/or the triplex.

Similarly, in case the first, second and third nucleic acid strands used in the method comprises a respective blocking nucleic acid strand, the blocking nucleic acid strand may preferably be introduced into the biological sample with the respective first, second and third nucleic acid strands. The method may preferably further comprise a step of removing or digesting the blocking nucleic acid strand to enable hybridisation to form the duplex and/or the triplex.

Generally, a plurality of markers may be introduced into the biological sample, the markers being specific to respective target analytes or pairs of target analytes, in order to identify a large number of (different or the same) target analytes at the same time. Preferably, a target analyte is identified and/or localised within the biological sample based on the label(s) of the marker(s), in particular the labelling moieties, associated with the target analyte in the optical readout.

The method has the same advantages as the label or marker. Further, the method may be supplemented with the features of the label or marker described in this document, in particular, the features of the dependent claims of the label or marker.

### Short Description of the Figures

Hereinafter, specific embodiments are described referring to the drawings, wherein:
- Figure 1: is a schematic view of a marker with a label for analysing a biological sample,
- Figure 2: is a schematic view of the marker according to Fig. 1 with the label comprising a triplex,
- Figure 3: is a schematic view of a first label part,
- Figure 4: is a schematic view of a marker comprising the first label part according to Fig. 3,
- Figure 5: is a schematic view of a marker according to a further embodiment, and
- Figure 6: is a schematic view of labels according to further embodiments.

### Detailed Description

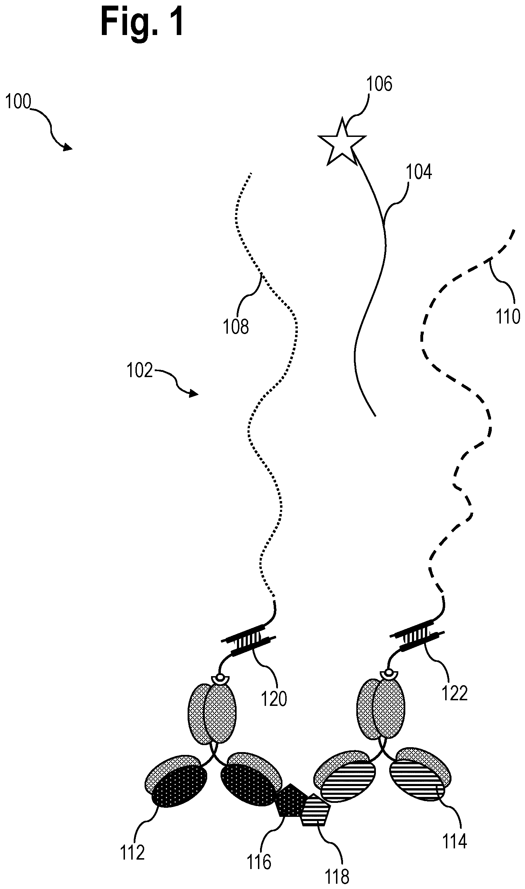

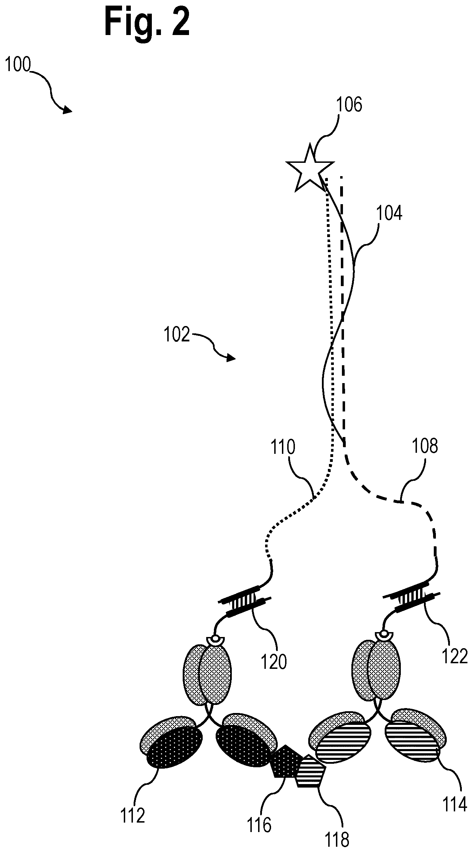

Figures 1 and 2 are schematic views of a marker 100 with a label 102 for analysing a biological sample. The label 102 comprises a first label part with a first nucleic acid strand 104 and at least one labelling moiety 106. The label 102 further comprises a second nucleic acid strand 108 and a third nucleic acid strand 110. The first, second and third nucleic acid strands 104, 108, 110 are configured to form a triplex. In particular, the second and third nucleic acid strands 108, 110 are configured to form a duplex and the first nucleic acid strand 104 is configured to hybridise to the duplex in order to form the triplex.

Figure 1 schematically shows the marker 100 with the first, second and third nucleic acid strands 104, 108, 110 separate from each other and not hybridised to each other. In contrast, Figure 2 schematically shows the marker 100 with the first, second and third nucleic acid strands 104, 108, 110 hybridised to each other and forming the triplex.

Specifically, the second and third nucleic acid strands 108, 110 may have respective sequences that are complementary to each other, in particular over at least a majority of their length. In particular, the second and third nucleic acid strands 108, 110 may be entirely complementary to each other. The complementarity may be based on Watson and Crick base-pairing, for example, comprising base pairs of adenine and thymine, and cytosine and guanine. The duplex formed between the second and third nucleic acid strands 108, 110 can be a double helix, in particular in a B-form.

The first nucleic acid strand 104 may have a respective sequence that is complementary to the duplex of the second and third nucleic acid strands 108, 110, in particular over at least a majority of the length of the sequence. In particular, the sequence of the first nucleic acid strand 104 may be entirely complementary to the duplex of the second and third nucleic acid strands 108, 110. The complementarity may be based on Hoogsteen base-pairing, for example, comprising base pairs of adenine and thymine, and cytosine and guanine. In particular, the hybridisation of the first nucleic acid strand 104 to the duplex of the second and third nucleic acid strands 108, 110 is not based on Watson and Crick base-pairing. The triplex formed between the first, second and third nucleic acid strands 104, 108, 110 of the label 102 can be a triple helix. In particular, the first nucleic acid strand 104 may be arranged along the major groove of the duplex between the second and third nucleic acid strands 108, 110.

The labelling moiety 106 may be a fluorophore, such as a fluorescent protein, an organic or inorganic fluorescent molecule, or a fluorescent nanoparticle. The labelling moiety 106 enables detecting the marker 100, in particular the label 102 with the triplex formed.

The marker 100 further comprises a first marker part comprising the second nucleic acid strand 108 and a first affinity reagent 112. Further, the marker 100 comprises a second marker part comprising the third nucleic acid strand 110 and a second affinity reagent 114. The affinity reagents 112, 114 are each configured to bind specifically to a target analyte 116, 118 of a biological sample. To that end, the affinity reagents 112, 114 may have a high affinity specifically for the respective target analyte 116, 118. Binding of the affinity reagent 112, 114 to the respective target analyte 116, 118 may be based on intermolecular forces such as hydrogen bonding and van der Waals forces. The affinity reagents 112, 114 can be one of an antibody, an antibody fragment, an aptamer, or a linear or primary nucleic acid, for example. The target analytes 116, 118 may be proteins that interact with each other, for example.

In the examples according to Figs. 1 and 2 the affinity reagents 112, 114 are shown as antibodies. Each of the affinity reagents 112, 114 is specifically bound to a respective one of the target analytes 116, 118.

In an alternative embodiment, the affinity reagents 112, 114 may be bound to the same target analyte, but be specific to different regions or epitopes of that same target analyte.

The second and third nucleic acid strands 108, 110 are bound to either the first affinity reagent 112 or the second affinity reagent 114, respectively. In particular, the second and third nucleic acid strands 108, 110 are bound to the respective affinity reagent 112, 114 via barcode oligonucleotides 120, 122 of the affinity reagents 112, 114, which are partially complementary to the respective second or third nucleic acid strands 108, 110. Alternatively, at least one of the second and third nucleic acid strands 108, 110 may be directly attached to the respective affinity reagent 112, 114.

When the first, second and third nucleic acid strands 104, 108, 110 are bound to each other to form the triplex, as shown in Fig. 2, the labelling moiety 106 is associated with the marker 100 and, via the affinity reagents 112, 114, with the target analytes 116, 118.

The duplex of the second nucleic acid strand 108 and the third nucleic acid strand 110 may only form, when the target analytes 116, 118 are in such proximity that the second and third nucleic acid strands 108, 110 may hybridise to each other.

Similarly, the triplex of the first, second and third nucleic acid strands 104, 108, 110 may only form, when the second and third nucleic acid strands 108, 110 have already hybridised to each other. Thus, also the triplex may only form, when the target analytes 116, 118 are in close proximity.

Thus, upon triplex formation, the association of the labelling moiety 106 of the first label part with the target analytes 116, 118 enables determining that the target analytes 116, 118 are in close proximity.

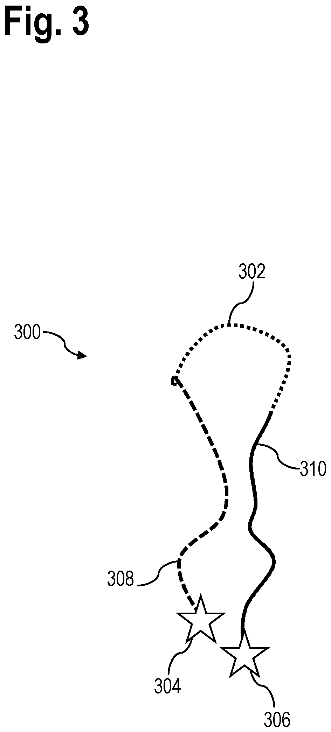

Figure 3 is a schematic view of a first label part 300 comprising a first nucleic acid strand 302, a first labelling moiety 304 and a second labelling moiety 306. The first nucleic acid strand 302 has a first sequence part 308 and a second sequence part 310. The first sequence part 308 is (at least partially) a reverse complement to the second sequence part 310. Thus, the first label part 300 forms a hairpin, as shown in Fig. 3. The first and second labelling moieties 304, 306 are arranged at respective ends of the first nucleic acid strand 302. Thus, when the first label part 300 has its hairpin conformation, the first and second labelling moieties 304, 306 are in close proximity, in particular within a Förster distance.

The first and second labelling moieties 304, 306 are configured for non-radiative energy transfer between them. For example, the labelling moieties 304, 306 may be a FRET pair or one of the first and second labelling moieties 304, 306 may be a quencher that quenches the fluorescent emission of the other one of the first and second labelling moieties 304, 306. Thus, when the first label part 300 has the hairpin conformation, the non-radiative energy transfer is possible between the first and second labelling moieties 304, 306.

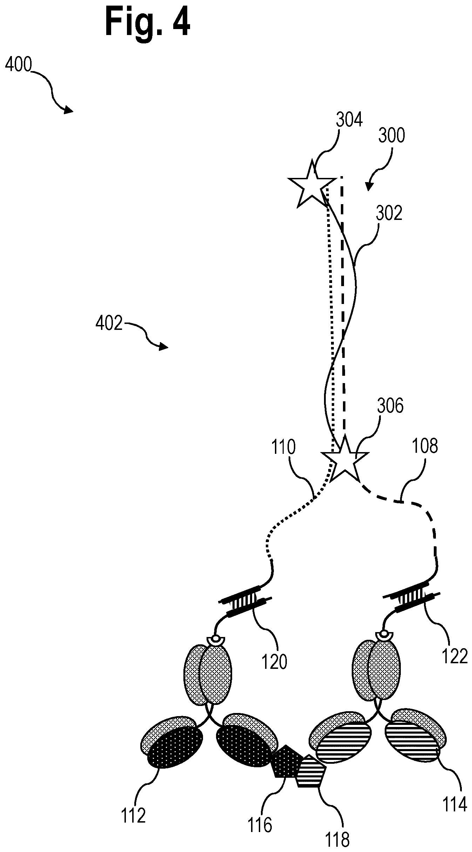

Figure 4 is a schematic view of a marker 400. The marker 400 comprises a label 402 with the first label part 300. The first nucleic acid strand 302 of the label part 300 is configured to form the triplex by hybridising to the duplex of the second and third nucleic acid strands 108, 110. In particular, the hybridised first sequence part 308 and second sequence part 310 may have a lower melting temperature than the triplex of the first nucleic acid strand 302 with the second and third nucleic acid strands 108, 110, such that the first nucleic strand 302 preferably forms the triplex, rather than the hairpin.

Upon hybridising to the duplex and therefore triplex formation, the first nucleic acid strand 302 is aligned in a linear or elongated conformation with the duplex. This separates the first and second labelling moieties 304, 306 essentially by the length of the first nucleic acid strand 302. Thus, the first and second labelling moieties 304, 306 are at an increased distance from each other compared to the hairpin conformation of the first label part 300. This increased distance is greater than the close proximity that the first and second labelling moiety 304, 306 are in when the first label part 300 has the hairpin conformation. Thus, when the first label part 300 is bound to the duplex, as shown in Fig. 3, the non-radiative energy transfer is not possible between the labelling moieties 304, 306. This enables efficiently and unambiguously distinguishing between the state of the marker 400 in which the triplex is not formed and the first label part 300 has its hairpin conformation and the state of the marker 400 in which the triplex is formed and the first label part 300 is elongated by hybridising along the second and third nucleic acid strands 108, 110. This distinguishing is particularly based on the difference in detectable optical properties of the labelling moieties 304, 306 with reference to whether the non-radiative energy transfer is occurring between the labelling moieties 304, 306.

Thus, if the labelling moieties 304, 306 can be individually detected, for example, by means of a fluorescence microscope, the first label part 300 is hybridised to the duplex of the second and third nucleic acid strands 108, 110 and consequently, the target analytes 116, 118 must be in close proximity.

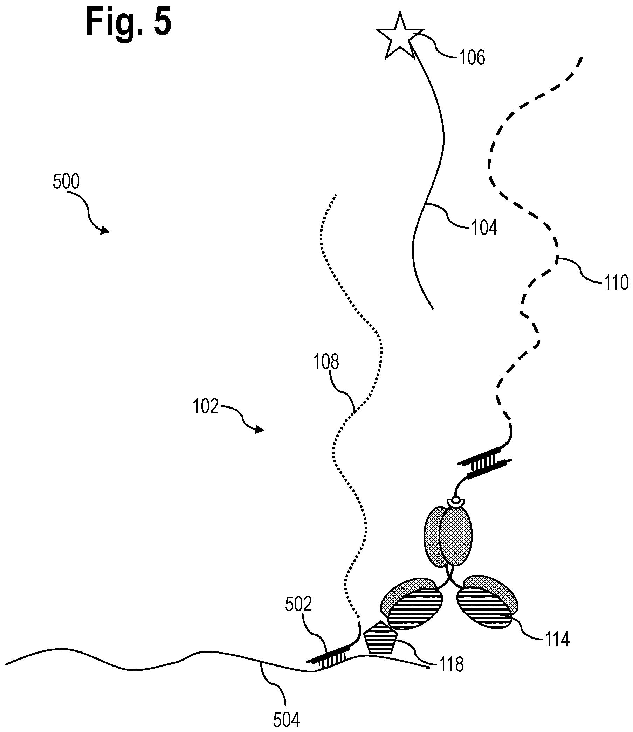

Figure 5 is a schematic view of a marker 500 comprising the label 102. In contrast to the marker 100 described above, the marker 500 comprises the second nucleic acid strand 108 attached to a nucleic acid affinity reagent 502. The nucleic acid affinity reagent 502 hybridises to a nucleic acid target analyte 504. The target analytes 118, 504 may interact with each other such that they are in close proximity. Thus, the marker 500 enables determining whether the target analytes 118, 504 are present and interacting with each other (or are in proximity to each other) based on the triplex formation between the first, second and third nucleic acid strands 104, 108, 110.

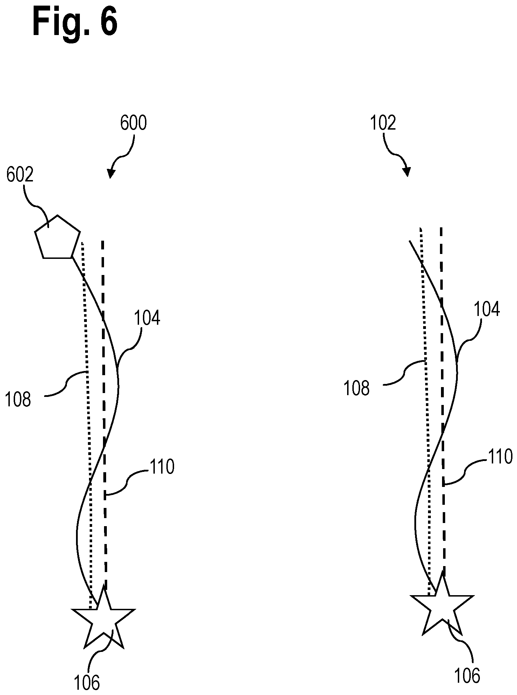

Figure 6 is a schematic view of a label 600 comprising an affinity moiety 602. The affinity moiety 602 is configured to specifically bind to a respective second affinity moiety (not shown) with high affinity. The affinity moiety 602 may be a biotin, streptavidin, adamantane or ferrocene, for example. The affinity moiety 602 enables immobilising or attaching the label 600 to a solid support, for example.

Thus, the affinity moiety 602 enables using the label 600 to capture as well as detect the second nucleic acid strand 108 and/or the third nucleic acid strand 110. In particular, this does not require the presence of affinity reagents or a marker, as described above. For example, the affinity moiety 602 may be captured on a stationary phase of a chromatography column by means of a corresponding second affinity moiety. For example, the affinity moiety 602 may be biotin and the stationary phase comprises streptavidin as the second affinity moiety.

In a particular embodiment, at least two of the first nucleic acid strand 104, the second nucleic acid strand 108, and the third nucleic acid strand 110 of the label 600 comprise an affinity moiety, in particular, the affinity moiety 602.

Alternatively, the label 600 may be used with the markers described above.

Figure 6 further shows schematically the label 102. Both, the labels 600, 102 may be used to detect endogenous nucleic acids of a biological sample. For example, at least one of the second and third nucleic acids 108, 110 may be an endogenous nucleic acid of a biological sample. In contrast, the other one of the second and third nucleic acids 108, 110 and the first nucleic acid 104 may be an exogenous nucleic acid.

When analysing a biological sample the labels 102, 600 or the markers 100, 400, 500 may be introduced into the biological sample. In particular, the respective first, second and third nucleic acids may be introduced separately and/or sequentially into the biological sample. In case at least one of the second and third nucleic acids 108, 110 is endogenous, only the exogenous nucleic acids are introduced into the biological sample.

The introduced first, second and third nucleic acid strands 104, 108, 110 may form the triplex in situ of the biological sample, in case the respective target analytes are present and in proximity within the biological sample. Further, the introduced labels 102, 600 or the markers 100, 400, 500 may bind to their respective target analytes.

In case at least one of the second and third nucleic acids 108, 110 being endogenous, the endogenous nucleic acid strands may be considered target analytes, which presence and/or proximity may be determined.

Subsequently, an optical readout may be generated of the biological sample with the introduced labels 102, 600 or markers 100, 400, 500. The labelling moieties 106, 304, 306 enable detecting the presence and/or location of the respective target analytes in the optical readout.

Optionally, the affinity moiety 602 of the label 600 enables extracting the label 600 with any endogenous nucleic acids from the biological sample in a subsequent step.

In order to avoid hybridisation and duplex or triplex formation of the first, second and third nucleic acid strands 104, 108, 110 prior to introduction into the biological sample, at least one of the first, second and third nucleic acid strands 104, 108, 110 may be brought into contact with blocking nucleic acid strands (not shown). The blocking nucleic acid strands maybe at least partially complementary to one of the first, second and third nucleic acid strands 104, 108, 110. After introducing the first, second and third nucleic acid strands 104, 108, 110 into the biological sample, the blocking strands may be removed in order to allow duplex and triplex formation. The blocking strands may be nuclease sensitive and removed by addition of a respective nuclease.

Alternatively, at least one of the first, second and third nucleic acid strand 104, 108, 110 may be provided with at least one guest molecule. These first, second and third nucleic acid strand 104, 108, 110 may be introduced into the biological sample in the presence of a respective host molecule. After introduction of the first, second and third nucleic acid strand 104, 108, 110 the host molecule may be removed, for example by addition of a competitive guest molecule, in order to allow duplex and triplex formation.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100, 400, 500: Marker
- 102, 402, 600: Label
- 104, 302: First nucleic acid strand
- 106, 304, 306: Labelling moiety
- 108: Second nucleic acid strand
- 110: Third nucleic acid strand
- 112, 114, 502: Affinity reagent
- 116, 118, 504: Target analyte
- 120, 122: Barcode oligonucleotide
- 300: First label part
- 308: First sequence part
- 310: Second sequence part
- 602: Affinity moiety

## Claims

1. A label (102, 402, 600) for analysing a biological sample comprising:
a first label part (300) comprising at least one first nucleic acid strand (104, 302),
a second label part comprising a second nucleic acid strand (108),
a third label part comprising a third nucleic acid strand (110),
wherein the first nucleic acid strand (104, 302), the second nucleic acid strand (108), and the third nucleic acid strand (110) are configured to form a triplex with each other, and
wherein the first label part (300) comprises at least one labelling moiety (106, 304, 306).

2. The label according to claim 1, where the second nucleic acid strand (108) and the third nucleic acid strand (110) are at least partially complementary to each other.

3. The label according to one of the preceding claims, wherein the at least one first nucleic acid strand (104, 302) is at least partially complementary to the hybridised second nucleic acid strand (108) and third nucleic acid strand (110).

4. The label according to one of the preceding claims, wherein the at least one first nucleic acid strand (104, 302) is a nucleic acid analogue.

5. The label according to one of the preceding claims, wherein at least one of the first nucleic acid strand (104, 302), the second nucleic acid strand (108), and the third nucleic acid strand (110) is a nuclease-resistant nucleic acid and/or wherein at least one of the other of the first nucleic acid strand (104, 302), the second nucleic acid strand (108), and the third nucleic acid strand (110) is a nuclease-sensitive nucleic acid.

6. The label according to one of the preceding claims comprising a plurality of first nucleic acid strands (104, 302) that each bind to a different part of the hybridised second nucleic acid strand (108) and third nucleic acid strand (110).

7. The label according to one of the preceding claims, wherein the at least one first nucleic acid strand (104, 302) comprises an affinity moiety (602).

8. The label according to one of the preceding claims, wherein the at least one first nucleic acid strand (104, 302) is immobilised on a solid support.

9. The label according to one of the preceding claims, wherein the at least one first nucleic acid strand (104, 302) is configured to form a secondary structure.

10. The label according to one of the preceding claims further comprising at least one guest molecule configured to form a complex with a host molecule.

11. The label according to one of the preceding claims further comprising at least one blocking nucleic acid strand at least partially complementary to one of the second nucleic acid strand (108) and the third nucleic acid strand (110).

12. A marker (100, 400, 500) for analysing a biological sample with a plurality of target analytes comprising:
a label (102, 402, 600) according to one of the preceding claims comprising a first label part (300), a second label part and a third label part,
a first marker part comprising a first affinity reagent (112, 502) and the second label part, and
a second marker part comprising a second affinity reagent (114) and the third label part, and
wherein the first affinity reagent (112, 502) and the second affinity reagent (114) are each configured to bind specifically to one of the target analytes (116, 118, 504) of the biological sample.

13. A method for analysing a biological sample, the method comprising the following steps:
introducing at least a first label part (300) into the biological sample,
wherein the first label part comprises at least one first nucleic acid strand (104, 302) and at least one labelling moiety (106, 304, 306).
wherein the first nucleic acid strand (104, 302) is configured to form a triplex with a second nucleic acid strand (108) and a third nucleic acid strand (110).
generating an optical readout of the biological sample with at least the first label part (302).

14. The method according to claim 13, wherein at least one of the second nucleic acid strand (108) and the third nucleic acid strand (110) are an endogenous nucleic acid of the biological sample.

15. The method according to one of the preceding claims 13 to 14, wherein one of the second nucleic acid strand (108) and the third nucleic acid strand (110) are introduced into the biological sample.

16. The method according to claim 13, wherein a second label part comprises the second nucleic acid strand (108) and a third label part comprises the third nucleic acid strand (110), and wherein the first label part (300), the second label part and the third label part are of a marker according to claim 12, and wherein the marker is introduced into the biological sample.

17. The method according to one of the preceding claims 13 to 16, comprising the steps of removing the first label part (300) from the second nucleic acid strand (108) and third nucleic acid strand (110), and of introducing a further first label part with a further first nucleic acid strand configured to form a triplex with the second nucleic acid strand (108) and the third nucleic acid strand (110).
